# EUROPÄISCHE PATENTSCHRIFT

(11) **EP 3 461 515 B1**
(45) Veröffentlichungstag und Bekanntmachung des Hinweises auf die Patenterteilung: **24.02.2021**
(21) Anmeldenummer: 18196481.8
(22) Anmeldetag: 25.09.2018
(51) Int. Cl.: A61M 1/16

(54) **MEHRFACHSENSORVORRICHTUNG**
MULTIPLE SENSOR ASSEMBLY
DISPOSITIF COMPRENANT PLUSIEURS MULTICAPTEUR

(30) Priorität: 28.09.2017 DE 102017122540
(43) Veröffentlichungstag der Anmeldung: 03.04.2019
(73) Patentinhaber: B. Braun Avitum AG, 34212 Melsungen (DE)
(72) Erfinder: JANIK, Waldemar, 34212 Melsungen (DE)
(74) Vertreter: Winter, Brandl, Fürniss, Hübner, Röss, Kaiser, Polte - Partnerschaft mbB

(56) Entgegenhaltungen:
- EP-A2- 0 898 974
- EP-A2- 0 980 686
- DE-A1-102014 111 732
- US-A1- 2011 009 800
- US-B1- 6 702 774

## Beschreibung

Die Erfindung betrifft eine Mehrfachsensorvorrichtung für ein medizinisches Gerät, und bezieht sich insbesondere auf eine mehrere individuelle Sensoren beinhaltende Mehrfachsensorvorrichtung oder Multisensorvorrichtung an einem Ausgang für Dialysierflüssigkeit, insbesondere verbrauchter Dialysierflüssigkeit, eines Dialysators einer Vorrichtung zur extrakorporalen Blutbehandlung, wie beispielsweise einer Dialysemaschine.

Bei einer extrakorporalen Blutbehandlung (z. B. einer Hämodialyse) wird Blut des Patienten von Dialysierflüssigkeit in einem Dialysator umspült. Zur Bestimmung beispielsweise des Drucks, der Temperatur, der Flussrate der Dialysierflüssigkeit, eines möglichen Blutlecks, der Leitfähigkeit, der Absorbanz bzw. Extinktion oder Fluoreszenz, von harnpflichtigen Substanzen und/oder Elektrolyten und/oder des pH-Werts beinhalten Dialysemaschinen häufig mehrere individuelle Sensoren am Ausgang für verbrauchte Dialysierflüssigkeit des Dialysators.

Manche dieser Sensoren bilden lediglich Sicherheitsvorkehrungen. Beispielsweise erfasst ein als Blutleckdetektor dienender Sensor im Fall einer Membranruptur Bluteinströmungen in die Dialysierflüssigkeit, die rechtzeitig erkannt werden müssen, um einen zu hohen Blutverlust des Patienten zu vermeiden. Andere Sensoren wiederum ermitteln eher unkritische Parameter wie beispielsweise die Dialysedosis Kt/V. Zur Ermittlung der Dialysedosis Kt/V werden beispielsweise optische Sensoren eingesetzt, die in der Dialysierflüssigkeit die Absorption von Licht messen, woraus sodann auf ein Vorhandensein harnpflichtiger Substanzen geschlossen werden kann. Andere Verfahren beruhen auf einer gleichzeitigen Leitfähigkeitsmessung dialysierflüssigkeitsseitig vor und hinter dem Dialysator.

Im Stand der Technik sind räumlich voneinander getrennt angeordnete optische Blutleckdetektoren und Leitfähigkeitssonden am Ausgang für verbrauchte Dialysierflüssigkeit bekannt. Ferner ist bekannt, zur Bestimmung der Dialysedosis Kt/V entweder einen optischen Sensor am Ausgang für verbrauchte Dialysierflüssigkeit oder Leitfähigkeitssonden sowohl am Eingang für frische Dialysierflüssigkeit als auch am Ausgang für verbrauchte Dialysierflüssigkeit anzuordnen. Die Druckschrift DE 10 20 14 012 423 A1 beispielsweise lehrt im Kontext eine Berücksichtigung von Flussraten zur Kombination zeitversetzter Signale räumlich getrennter Sensoren. Aus US2011009800 ist eine Mehrfachsensorvorrichtung für Dialysegeräte bekannt.

Die bekannten Lösungen sind jedoch insoweit nachteilig, als die einzelnen Sensoren jeweils ein eigenes Gehäuse sowie einen eigenen Kabelstrang aufweisen und Elemente zur Verbindung der einzelnen Sensoren, wie beispielsweise Schläuche, Schlaucholiven und dergleichen, erforderlich sind, woraus ein hoher Platzbedarf resultiert. Die bekannten Lösungen sind weiter nachteilig aufgrund der Ausbildung schwer zu desinfizierender Toträume zwischen den einzelnen Sensoren. Ferner müssen nachteilig Signalverluste aufgrund langer Strecken zwischen den einzelnen Sensoren und daraus resultierender Vermischung bzw. Verdünnung und Zeitverschiebung in Kauf genommen werden. Außerdem sind zur Korrektur zeitverschobener Signale aufwandserhöhend weitere Parameter wie Flussraten und Volumina zu berücksichtigen.

Der Erfindung liegt daher als eine Aufgabe zugrunde, eine Sensorvorrichtung zu schaffen, bei welcher sowohl optische als auch nicht-optische Sensoren am Ausgang für verbrauchte Dialysierflüssigkeit eines Dialysators so miteinander kombiniert sind, dass sie dort nur wenig Platz oder Raum beanspruchen und von ihnen gelieferte Daten und/oder Signale ohne Berücksichtigung insbesondere fluss- und volumenabhängiger Signalverschiebungen auswertbar sind.

Erfindungsgemäß wird diese Aufgabe durch eine Mehrfachsensorvorrichtung mit den Merkmalen des Anspruchs 1 gelöst. Vorteilhafte Weiterbildungen der Erfindung sind Gegenstand der beigefügten Unteransprüche.

Es wird demzufolge prinzipiell eine Mehrfachsensorvorrichtung für ein medizinisches Gerät vorgeschlagen, aufweisend zumindest eine erste Sensoreinrichtung und zumindest eine n-te Sensoreinrichtung, die an einer Fluid führenden Leitungsverbindung angeordnet sind,
wobei die Sensoreinrichtungen dazu eingerichtet sind, jeweils zumindest einen eine physikalische Größe des durch die Leitungsverbindung strömenden Fluids kennzeichnenden Messwert zu erfassen,
wobei die Sensoreinrichtungen derart in einem maximalen räumlichen Abstand zueinander angeordnet sind, dass die durch diese Sensoreinrichtungen erfassten Messwerte in einem vorbestimmten maximalen Zeitintervall erfassbar sind.

In anderen Worten ist die vorliegende Offenbarung auf eine Mehrfachsensorvorrichtung für ein medizinisches Gerät gerichtet, bei der zumindest eine erste Sensoreinrichtung und zumindest eine n-te Sensoreinrichtung an einer Fluid führenden Leitungsverbindung, entlang welcher die Sensoreinrichtungen zumindest eine Größe aus einem strömenden Fluid detektieren, in vorbestimmter räumlicher Nähe zueinander angeordnet sind, wobei eine maximale Distanz zwischen der ersten Sensoreinrichtung und der n-ten Sensoreinrichtung durch eine vorbestimmte maximale Zeitverzögerung zwischen der Erfassung einer ersten Signaländerung eines ersten Signals der ersten Sensoreinrichtung und der Erfassung einer zu der ersten Signaländerung korrespondierenden Signaländerung eines n-ten Signals der n-ten Sensoreinrichtung bei einer vorbestimmten Flussrate durch die Fluid führende Leitungsverbindung bestimmt ist, und die räumliche Nähe der ersten Sensoreinrichtung und der n-ten Sensoreinrichtung derart vorbestimmt ist, dass bei der vorbestimmten Flussrate in dem ersten Signal der ersten Sensoreinrichtung und in dem n-ten Signal der n-ten Sensoreinrichtung jeweils zueinander korrespondierende Signaländerungen bei parallel gleichzeitiger Erfassung beider Signale innerhalb der vorbestimmten maximalen Zeitverzögerung quasi zeitgleich auftreten.

Bei medizinischen Geräten wie beispielsweise einer Maschine für extrakorporale Blutbehandlung erfassen verschiedenartige Sensoreinrichtungen jeweils für sie typische Daten und/oder Signale und geben diese weiterverarbeitbar aus. Vor diesem allgemeinen Hintergrund sollen erfindungsgemäß Daten und/oder Signale der verschiedenartigen Sensoreinrichtungen fusioniert werden, um auf der Grundlage der Sensorausgaben weitere Parameter zu ermitteln oder zu berechnen. Hierbei sollen insbesondere schnelle Signaländerungen erfassbar sein, so dass Laufzeiten und/oder Verzögerungszeiten zwischen relevanten Teilen der Sensorausgaben zu minimieren sind, die andernfalls mit erhöhtem Aufwand und der Erfassung schneller Signaländerungen entgegenwirkend zu eliminieren wären. In anderen Worten beschleunigt und erleichtert der Entfall von strömungsgeschwindigkeitsbedingten Laufzeiten oder Verzögerungszeiten die Erfassung und Ermittlung eines gewünschten Signals oder Parameters. Damit spielen bei Anordnung der einzelnen Sensoren in unmittelbarer Nähe zueinander Laufzeiten und Verzögerungszeiten keine Rolle mehr.

Gemäß der zugrunde liegenden erfinderischen Idee werden dazu in einer Abführleitung für verbrauchte Dialysierflüssigkeit mehrere unterschiedliche Sensoren, genauer zumindest ein optischer Sensor und zumindest ein nicht-optischer Sensor, wobei der optische Sensor beispielsweise Daten und/oder Signale zur Ermittlung der Dialysedosis Kt/V liefern und der nicht-optische Sensor eine Leitfähigkeitszelle oder Leitfähigkeitssonde und eine Temperatursonde umfassen kann, in unmittelbarer Nähe zueinander angeordnet. Als unmittelbare Nähe wird hierbei ein maximaler Abstand bzw. eine maximale Distanz zwischen jeweils betrachteten Sensoren verstanden, bei dem beispielsweise (bei vorbestimmtem Fluss bzw. vorbestimmter Flussrate abgeführter Dialysierflüssigkeit) eine Zeitverzögerung zwischen Signalausgaben der betrachteten Sensoren eine vorbestimmte oder maximale bzw. tolerierbare Dauer nicht überschreitet.

Wenn beispielsweise der Innendurchmesser einer Abführleitung 5 mm beträgt, die Dialysierflüssigkeit mit einem Fluss von 500 ml/min durch die Abführleitung strömt, und an einer Dialysemaschine Flüsse zwischen 300 ml/min und 800 ml/min einstellbar sind, beträgt bei einem angenommenen mittleren Fluss von 500 ml/min in der Abführleitung eine maximale Distanz zwischen zwei Sensoren 424 mm, falls eine Zeitverzögerung bzgl. der Signale an einem ersten und einem zweiten Sensor von maximal 1 Sekunde tolerierbar sein soll. Die mögliche Distanz vergrößert sich mit zunehmendem Fluss durch die Abführleitung, und kann bei einem Fluss von 800 ml/min etwa 679 mm betragen. Beträgt die Zeitverzögerung maximal etwa 1 Sekunde, so befinden sich im Sinne der Erfindung die Sensoren in unmittelbarer Nähe zueinander. Bevorzugt kann daher eine maximale Distanz zwischen einem ersten Sensor und einem n-ten Sensor von 680 mm, wobei zwischen dem ersten und dem n-ten Sensor (n-2) weitere Sensoren platziert sein können, vorbestimmt werden. Optimalerweise sind darüber hinaus die Sensoren so nahe beieinander angeordnet, dass lange Schlauchverbindungen entfallen können.

In anderen Worten ist nicht der maximal einstellbare Dialysierflüssigkeitsfluss der limitierende Faktor für eine maximale Distanz, sondern der minimal einstellbare Fluss. Wenn man also von einer maximalen zeitlichen Verschiebung von 1 Sekunde (d. h. "quasi-zeitgleich" im Sinne der vorliegenden Offenbarung) ausgeht und der Innendurchmesser 5mm beträgt, dann beträgt die maximale Distanz
- 679 mm bei einem Fluss von 800 ml/min;
- 424 mm bei einem Fluss von 500 ml/min;
- 254 mm bei einem Fluss von 300 ml/min;
- 85 mm bei einem Fluss von 100 ml/min.

Bislang ist aus dem Stand der Technik zum einen eine Kombination von Messwerten der vorgenannten Sensoreinrichtungen bzw. Sensoren, d.h. eine Nutzung der entsprechenden Parameter zur Bestimmung eines anderen Parameters im Rahmen einer Messdatenfusion, nicht bekannt. In anderen Worten wird eine bislang nicht bekannte Kombination aus Leitfähigkeitssonde/Temperatursonde und optischem Sensor zur Bestimmung der Dialysedosis geschaffen. Zum anderen ist aus dem Stand der Technik die erfindungsgemäße Kombination der vorgenannten Sensoreinrichtungen bzw. Sensoren in einer Abführleitung für beispielsweise verbrauchte Dialysierflüssigkeit nicht bekannt. In anderen Worten können die einzelnen Sensoren bereits andernorts verbaut sein, ermöglichen in ihrer Position andernorts dann jedoch nicht die hier zugrunde liegende Erfassung und/oder Ermittlung schneller Signaländerungen ohne Berücksichtigung von Signalverzögerungszeiten.

Erfindungsgemäß resultieren somit eine vorteilhafte und unter anderem eine platzsparende Anordnung der Sensoren innerhalb der Maschine, der Entfall zeitversetzter Signale aufgrund der Aufhebung oder zumindest Verringerung der räumlichen Trennung in einem Maße derart, dass Flussraten und/oder Volumina nicht länger zu berücksichtigen sind, und eine kompakte Bauweise unter Vermeidung unerwünschter Toträume.

Im Einzelnen wird die Aufgabe gelöst durch eine Mehrfachsensorvorrichtung für ein medizinisches Gerät, bei der zumindest eine erste Sensoreinrichtung und zumindest eine n-te Sensoreinrichtung an einer Fluid führenden Leitungsverbindung, entlang welcher die Sensoreinrichtungen zumindest eine Größe aus einem strömenden Fluid detektieren, derart in vorbestimmter räumlicher Nähe zueinander angeordnet sind, dass vorbestimmte Signalanteile in Ausgaben jeweils der ersten und der n-ten Sensoreinrichtung praktisch zeitgleich, auftreten und erfassbar sind. Praktisch zeitgleich bedeutet vorzugsweise, dass die vorbestimmten Signalanteile eine vorbestimmte maximale Zeitverzögerung zwischen ihrem Auftreten nicht überschreiten. Vorteilhaft sind dadurch Signalanteile in den Ausgaben der einzelnen Sensoren, die Grundlage für eine Weiterverarbeitung und insbesondere eine Messdatenfusion zur Bestimmung eines daraus ermittelbaren weiteren Parameters bilden, hinreichend verzögerungsfrei bzw. zeitgleich erfassbar, und eine zeitaufwendige rechnerische Eliminierung von Verzögerungszeiten und/oder Laufzeiten kann entfallen. Es wird angemerkt, dass die detektierte Größe vorzugsweise eine physikalische Größe sein kann.

Erfindungsgemäß wird hierbei eine maximale Distanz zwischen der ersten Sensoreinrichtung und der n-ten Sensoreinrichtung durch eine vorbestimmte maximale Zeitverzögerung von einer Sekunde zwischen der Erfassung eines Signals der ersten Sensoreinrichtung und der Erfassung eines Signals der n-ten Sensoreinrichtung bei einer vorbestimmten Flussrate im Bereich zwischen 100 ml/min und 800 ml/min durch die Fluid führende Leitungsverbindung bestimmt. Da die maximale Distanz von einer jeweiligen Flussrate durch die Fluid führende Leitungsverbindung abhängig ist, kann bei bekannter Flussrate durch Vorgabe einer Zeitbedingung, beispielsweise einer maximalen Zeitverzögerung bezüglich der Signale an der ersten Sensoreinrichtung und der n-ten Sensoreinrichtung, eine maximale Distanz zwischen der ersten und einer jeweils n-ten Sensoreinrichtung ermittelt bzw. festgelegt werden.

Bevorzugt istdas medizinische Gerät eine Maschine für extrakorporale Blutbehandlung; ist die zumindest eine erste Sensoreinrichtung eine nicht-optische Sensoreinrichtung; ist die zumindest eine n-te Sensoreinrichtung eine optische Sensoreinrichtung; und ist die Fluid führende Leitungsverbindung eine Abführleitung für verbrauchte Dialysierflüssigkeit der Maschine für extrakorporale Blutbehandlung. Vorteilhaft eignet sich eine solche Kombination von individuell und verzögerungsfrei erfassenden Sensoreinrichtungen in einer Maschine für extrakorporale Blutbehandlung, beispielsweise einer Dialysemaschine, für eine bevorzugte Messdatenkombination.

Bevorzugt ist bei der Mehrfachsensorvorrichtung eine Gehäuseeinrichtung an der Abführleitung für verbrauchte Dialysierflüssigkeit dazu angeordnet, die zumindest eine nicht-optische Sensoreinrichtung und die zumindest eine n-te optische Sensoreinrichtung in der vorbestimmten räumlichen Nähe zueinander aufzunehmen; ist die zumindest eine nicht-optische Sensoreinrichtung dazu angeordnet, ein erstes Signal aus einer in der Abführleitung für verbrauchte Dialysierflüssigkeit strömenden verbrauchten Dialysierflüssigkeit zu erfassen und auszugeben; ist die zumindest eine n-te optische Sensoreinrichtung dazu angeordnet, ein n-tes Signal aus der in der Abführleitung für verbrauchte Dialysierflüssigkeit strömenden verbrauchten Dialysierflüssigkeit zu erfassen und auszugeben; wobei die räumliche Nähe der ersten Sensoreinrichtung und der zweiten Sensoreinrichtung entlang der Abführleitung für verbrauchte Dialysierflüssigkeit derart vorbestimmt ist, dass in dem ersten Signal der ersten Sensoreinrichtung und in dem zweiten Signal der zweiten Sensoreinrichtung jeweils zueinander korrespondierende Signalabschnitte bei parallel gleichzeitiger Erfassung beider Signale praktisch zeitverschiebungsfrei bzw. praktisch zeitgleich auftreten, d.h. eine vorbestimmte maximale Zeitverzögerung nicht überschreiten. Vorteilhaft erlaubt die Anordnung der unterschiedlichen Sensoreinrichtungen in einem gemeinsamen Gehäuse eine modulartige Bauweise und Freiheitsgrade in Bezug auf eine optimierte Positionierung der Sensoren. Da es sich bei den Sensoreinrichtungen jeweils um fluiddurchflossene Sensoreinrichtungen oder nicht fluiddurchflossene Sensoreinrichtungen handeln kann, können die einzelnen Sensoreinrichtungen als modulartige Komponenten ausgebildet sein und sind kürzestmögliche Leitungsführungen, beispielsweise unter Verwendung kurz bauender Steckverbindungen oder Fluiddurchleitungen, zwischen den einzelnen Sensoreinrichtungen innerhalb der Gehäuseeinrichtung und damit größtmögliche Nähe der einzelnen Sensoreinrichtungen zueinander darstellbar.

Bevorzugt beinhaltet bei der Mehrfachsensorvorrichtung die zumindest eine erste Sensoreinrichtung eine Leitfähigkeitszelle zur Bestimmung einer Leitfähigkeit verbrauchter Dialysierflüssigkeit und eine Temperatursonde zur Messung einer Temperatur verbrauchter Dialysierflüssigkeit und zur Temperaturkompensation der Leitfähigkeitszelle; und beinhaltet die zumindest eine zweite Sensoreinrichtung zumindest einen optischen Sensor in Form zumindest einer Fotodiode und vorzugsweise zwei Fotodetektoren zur Bestimmung einer Absorptionseigenschaft der verbrauchten Dialysierflüssigkeit. Vorteilhaft eignet sich eine Kombination der von diesen Sensoreinrichtungen ausgegebenen Messdaten für eine bevorzugte Messdatenfusionierung.

Bevorzugt sind bei der Mehrfachsensorvorrichtung die Leitfähigkeitszelle und die Temperatursonde zu einer gemeinsamen Einheit innerhalb der Vorrichtung zusammengefasst und verbaut. Vorteilhaft kann hierdurch eine Vorverdichtung von in größtmöglicher Nähe zueinander zu verbauender Komponenten realisiert werden.

Bevorzugt ist bei der Mehrfachsensorvorrichtung eine unmittelbare Nähe der Temperatursonde zu dem optischen Sensor derart vorbestimmt, dass eine von der Temperatursonde erfasste Temperatur zur Korrektur temperaturabhängiger Effekte in dem bzw. innerhalb des optischen Sensor(s) heranziehbar ist.

Bevorzugt ist bei der Mehrfachsensorvorrichtung die vorbestimmte räumliche Nähe derart festgelegt, dass innerhalb der Mehrfachsensorvorrichtung die jeweiligen Sensoreinrichtungen zumindest untereinander schlauchverbindungsfrei angeordnet und aufgenommen sind.

Bevorzugt ist bei der Mehrfachsensorvorrichtung der optische Sensor unmittelbar vor der Leitfähigkeitszelle oder unmittelbar nach der Temperatursonde angeordnet, und stärker bevorzugt vor der Temperatursonde angeordnet.
Bevorzugt ist bei der Mehrfachsensorvorrichtung der optische Sensor mit einem Blutleckdetektor kombiniert angeordnet.

Erfindungsgemäß wird die Aufgabe auch gelöst durch ein nicht beanspruchtes Verfahren zur Festlegung einer räumlichen Nähe bei einer wie vorstehend angegebenen Mehrfachsensorvorrichtung, bei dem Positionen der einzelnen Sensoren in der Mehrfachsensorvorrichtung variiert werden und jeweils das Auftreten vorbestimmter Signalanteile in zumindest zwei von den einzelnen Sensoren detektierten Signalen erfasst wird; und diejenigen Positionen als Positionen der räumlichen Nähe festgelegt werden, bei welchen die vorbestimmten Signalanteile praktisch zeitgleich auftreten. Die Erfindung wird nachstehend unter Bezugnahme auf die beigefügte Zeichnung näher beschrieben. Es zeigen:
Fig. 1 eine schematische Darstellung einer Mehrfachsensorvorrichtung gemäß einem Ausführungsbeispiel; und
Fig. 2 eine schematische Darstellung, die linksseitig einen zeitlichen Verlauf zweier Signale eines ersten Sensors und eines zweiten Sensors, deren räumliche Anordnung zu einer Zeitverschiebung zwischen Extrema der einzelnen Sensorsignale führt, darstellt, und rechtsseitig einen zeitlichen Verlauf zweier Signale des ersten und des zweiten Sensors mit erfindungsgemäß räumlicher Nähe der Sensoren derart, dass Extrema der einzelnen Sensorsignale zeitgleich erscheinen, darstellt.

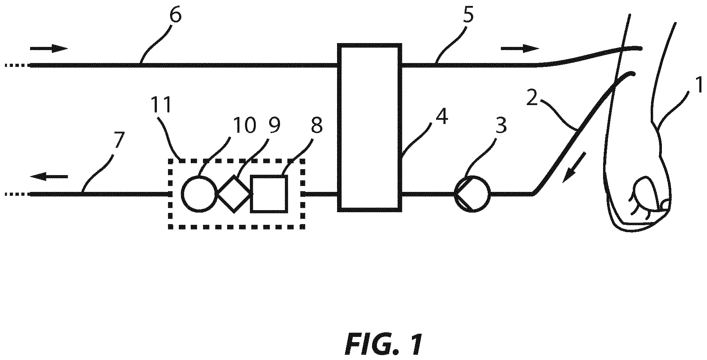

In Übereinstimmung mit Fig. 1 und wie in dieser vereinfacht dargestellt, wird im Rahmen einer extrakorporalen Blutbehandlung einem Patienten 1 über ein arterielles Schlauchsystem 2 mithilfe einer Fördereinrichtung 3 Blut entnommen und zu einem Dialysator 4 gefördert. In dem Dialysator 4 wird das Blut des Patienten von harnpflichtigen Substanzen und überschüssigem Wasser befreit. Anschließend wird das gereinigte Blut über ein venöses Schlauchsystem 5 dem Patienten zurückgegeben. Die Entnahme und Rückgabe des Bluts über eine gemeinsame Kanüle ist ebenfalls denkbar.

Es wird angemerkt, dass in Fig. 1 an sich bekannte Komponenten und Bestandteile einer in diesem Ausführungsbeispiel zur näheren Beschreibung herangezogenen Maschine für eine extrakorporale Blutbehandlung, wie beispielsweise Einheiten zur Proportionierung der Dialysierflüssigkeit oder zur Bilanzierung oder sonstige Komponenten, die grundlegende Funktionen einer üblichen Maschine zur extrakorporalen Blutbehandlung übernehmen, nicht dargestellt sind und hierein nicht beschrieben werden.

Im Dialysator 4 befinden sich Hohlfaserkapillaren (nicht gezeigt), die eine semipermeable Membran (nicht gezeigt) aufweisen. Die Kapillaren werden von der sogenannten Dialysierflüssigkeit umspült, die zum einen harnpflichtige Stoffe und überschüssiges Wasser aus dem Blut aufnimmt und zum anderen insbesondere Hydrogenkarbonat zur Behandlung einer Azidose des Patienten 1 abgibt.

Die Dialysierflüssigkeit strömt durch eine Zuführleitung 6 in den Dialysator 4. Ein Ausgang für verbrauchte Dialysierflüssigkeit 4a des Dialysators 4 mündet in eine Abführleitung 7, in deren weiterem Verlauf zumindest ein nicht-optischer Sensor 8, 9 und zumindest ein optischer Sensor 10 angeordnet sind.

Der zumindest eine nicht-optische Sensor 8, 9 umfasst in dem vorliegenden Ausführungsbeispiel beispielsweise eine Leitfähigkeitszelle 8 zur Bestimmung der Leitfähigkeit verbrauchter Dialysierflüssigkeit und eine Temperatursonde 9 zur Messung der Temperatur verbrauchter Dialysierflüssigkeit und zur Temperaturkompensation der Leitfähigkeitszelle 8.

Der zumindest eine optische Sensor 10 umfasst in dem vorliegenden Ausführungsbeispiel beispielsweise zumindest eine Fotodiode und vorzugsweise zwei Fotodetektoren, und wird zur Bestimmung einer Absorptionseigenschaft der verbrauchten Dialysierflüssigkeit eingesetzt.

Dabei handelt es sich bevorzugt um die Absorbanz bzw. Extinktion, die dann gemessen werden kann, wenn die verbrauchte Dialysierflüssigkeit Substanzen aufweist, die Licht absorbieren. Die Fotodiode des optischen Sensors 10 emittiert hierzu schmalbandiges Licht im UV-Bereich mit Wellenlängen vorzugsweise zwischen 200 und 350 nm. Weiter vorzugsweise wird Licht mit einer Peak-Wellenlänge zwischen 275 und 295 nm emittiert. Die Erfindung ist nicht auf diese konkreten Anzahlen von Sensoren und Wellenlängen verwendeten Lichts beschränkt. Auch das Emittieren und Detektieren mehrerer Wellenlängen ist von der Erfindung umfasst.

Alternativ kann der optische Sensor 10 dazu eingerichtet sein, Licht zum Anregen optisch aktiver Substanzen zu emittieren und anschließend Fluoreszenzemissionen zu messen. Weiter alternativ kann der optische Sensor 10 auf einer laserinduzierten Plasmaspektroskopie basieren.

Wie in Fig. 1 genauer dargestellt ist, sind in dem vorliegenden Ausführungsbeispiel der Mehrfachsensorvorrichtung die vorgenannten Sensoren 8, 9 und 10 in einem gemeinsamen Gehäuse bzw. einer Gehäuseeinheit 11 aufgenommen und derart angeordnet, dass sie sich in unmittelbarer Nähe zueinander befinden und vorzugsweise eine kompakte Einheit ausbilden. Innerhalb der Mehrfachsensorvorrichtung können speziell die Leitfähigkeitszelle 8 und die Temperatursonde 9 weiter zu einer gemeinsamen Einheit zusammengefasst und verbaut sein.

In dem vorliegenden Ausführungsbeispiel kann ein Innendurchmesser der Abführleitung 7 beispielsweise 5 mm betragen, und kann verbrauchte Dialysierflüssigkeit mit einem Fluss von beispielsweise 500 ml/min durch diese Abführleitung 7 strömen. Es wird angemerkt, dass die vorgenannten Werte praxisnahe Werte aus einem Bereich darstellen, die bei einer bekannten Dialysemaschine realisiert und/oder einstellbar sind. Beispielsweise lassen sich an einer bekannten Dialysemaschine Flüsse frischer bzw. verbrauchter Dialysierflüssigkeit zwischen 300 ml/min oder weniger und 800 ml/min einstellen. Ein Fluss von 500 ml/min stellt insoweit einen mittleren Wert im Einstellbereich der bekannten Dialysemaschine dar.

In dem vorliegenden Ausführungsbeispiel soll darüber hinaus eine Zeitverzögerung von beispielsweise maximal 1 Sekunde bezüglich der bzw. zwischen den Signalen an einem ersten Sensor (beispielsweise dem Sensor 8) und einem zweiten Sensor (beispielsweise dem Sensor 10) tolerierbar sein. Unter der Annahme eines mittleren Flusses von 500 ml/min in der Abführleitung 7 beträgt damit rechnerisch eine maximale Distanz zwischen dem ersten und dem zweiten Sensor 424 mm. Es wird angemerkt, dass sich die maximale Distanz mit steigender Flussrate durch die Abführleitung 7 vergrößert und bei einem Fluss von 800 ml/min etwa 679 mm beträgt.

Mithin wird die erfindungsgemäß unmittelbare Nähe durch die maximal tolerierbare Zeitverzögerung (beispielsweise 1 Sekunde) zwischen zu betrachtenden Signalen zweier Sensoren (einem ersten und einem zweiten Sensor) gegeben. In anderen Worten befinden sich zwei Sensoren in unmittelbarer Nähe zueinander, wenn die Zeitverzögerung zwischen ihren Signalen maximal etwa 1 Sekunde beträgt.

In diesem Ausführungsbeispiel wird daher bevorzugt eine maximale Distanz zwischen einem ersten Sensor (beispielsweise dem Sensor 8) und einem n-ten Sensor (beispielsweise dem Sensor 10) von 680 mm (entsprechend der hier größten einstellbaren Flussrate vom 800 ml/min) festgelegt. Zwischen dem ersten Sensor und dem n-ten Sensor können sodann (n-2) weitere Sensoren (beispielsweise der Sensor 9) platziert sein, alternativ beispielsweise insbesondere auch gleichartige und damit mehrfach angeordnete Sensoren, die in diesem Fall für niedrigere Flussraten die Bedingung der unmittelbaren Nähe unter Einhaltung des Zeitkriteriums, wie vorstehend beschrieben, gewährleisten. Vorteilhaft sind diese n Sensoren so nahe zueinander angeordnet, dass lange Schlauchverbindungen entfallen.

Es versteht sich, dass die Erfindung nicht auf die vorstehend beispielhaft angegebenen Zahlenwerte und/oder Einstellbereiche beschränkt ist, sondern sich entsprechende Verhältnisse, Beziehungen und Größenordnungen auch für andere Durchmesser der Abführleitung 7 und/oder einstellbare Flüsse und/oder Distanzen bzw., zeitliche Bedingungen ergeben werden.

Durch die die einzelnen Sensoren 8, 9 und 10 in unmittelbarer Nähe zueinander aufweisende Mehrfachsensorvorrichtung gemäß dem vorliegenden Ausführungsbeispiel wird eine zuverlässige Kompensation eines Temperatureffekts bzw. Temperatureinflusses auf die Leitfähigkeitsmessung möglich, ohne Temperaturverluste oder zeitversetzte Signale berücksichtigen zu müssen.

Aufgrund der direkten bzw. unmittelbaren Nähe der Temperatursonde 9 zu dem optischen Sensor 10 kann darüber hinaus vorteilhaft die (erfasste) Temperatur zur Korrektur möglicher temperaturabhängiger Effekte in dem optischen Sensor 10 herangezogen werden.

Die Anordnung der Sensoren 8, 9 und 10 in räumlicher Nähe zueinander minimiert darüber hinaus vorteilhaft Effekte, die insbesondere aufgrund flussabhängiger, zeitversetzter Signale bzw. Signaländerungen auftreten. Dadurch sind Signale bzw. Signaländerungen im erfindungsgemäßen Rahmen quasi zeitgleich von den jeweiligen Sensoren 8, 9 und 10 erfassbar, und kann der durch die Abführleitung 7 strömende Fluss verbrauchter Dialysierflüssigkeit unbeachtlich bleiben, d.h. er spielt demnach keine weitere Rolle im Hinblick auf die zu erfassenden und zu verarbeitenden Signale. Dies ist insbesondere dann von Interesse, wenn Verfahren zur Bestimmung relevanter Parameter auf Sensorfusionsansätzen bzw. Sensorvereinigungsansätzen beruhen.

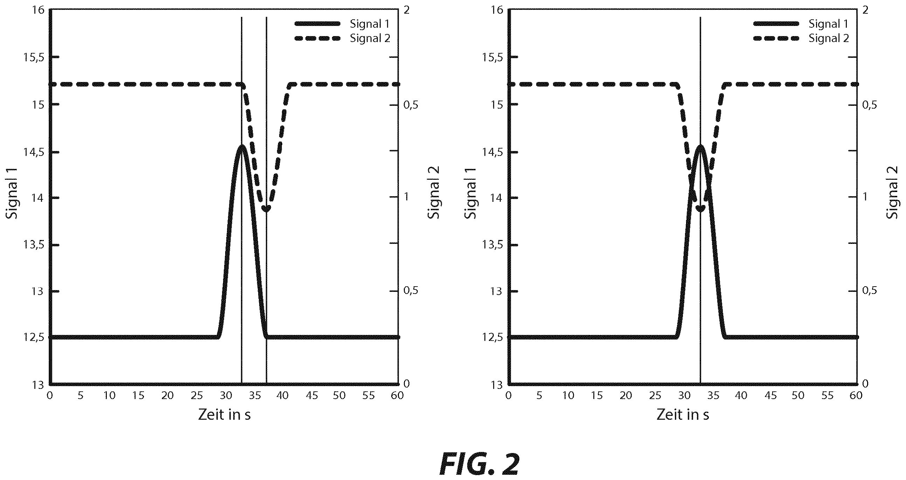

Zur qualitativen Veranschaulichung dieses Sachverhalts wird nachstehend auf Fig. 2 Bezug genommen. Links und rechts in Fig. 2 sind jeweils ein zeitlicher Verlauf zweier Signale dargestellt. Ein erstes Signal 1 entstammt einem ersten Sensor (beispielsweise dem nicht-optischen Sensor 8, 9 in dem vorliegenden Ausführungsbeispiel), und ein zweites Signal 2 entstammt einem zweiten Sensor (beispielsweise dem optischen Sensor 10 in dem vorliegenden Ausführungsbeispiel). Es versteht sich hierbei, dass das erste Signal 1 auch dem optischen Sensor 10 und das zweite Signal 2 auch dem nicht-optischen Sensor 8, 9 entstammen kann.

Wie in Fig. 2 linksseitig und als Vergleichsbeispiel einer bekannten Anordnung dargestellt ist, wird nach beispielsweise etwa 30 s Erfassungsdauer von dem ersten Sensor eine Signaländerung des ersten Signals 1 detektiert. Beispielhaft soll ein nach etwa 33 s auftretendes Maximum bzw. Extremum des ersten Signals 1 von Interesse sein. Ein Minimum bzw. Extremum in dem zweiten Signal 2 des zweiten Sensors tritt bezogen auf das Maximum des ersten Signals 1 erst 4 s später auf, d. h. die beiden Extrema des ersten Signals 1 und des zweiten Signals 2 werden zeitversetzt erfasst. Sind Flussrate und Volumen zwischen dem ersten und dem zweiten Sensor bekannt, kann daraus die Zeitverschiebung berechnet werden. Beide Extrema können dann miteinander fusioniert bzw. verschmolzen bzw. vereinigt oder zusammengeführt werden.

Rechtsseitig in Fig. 2 sind in Übereinstimmung mit der Mehrfachsensorvorrichtung gemäß dem vorliegenden Ausführungsbeispiel ebenfalls zwei Signalverläufe dargestellt. Auch in diesem Fall wird nach beispielsweise etwa 30 s Erfassungsdauer von dem ersten Sensor eine Signaländerung des ersten Signals 1 detektiert, und soll beispielhaft ein nach etwa 33 s auftretendes Maximum bzw. Extremum des ersten Signals 1 von Interesse sein. Unmittelbar ersichtlich tritt gemäß dem Ausführungsbeispiel ein Minimum bzw. Extremum in dem zweiten Signal 2 des zweiten Sensors bezogen auf das Maximum des ersten Signals 1 praktisch zeitgleich mit dem Maximum des ersten Signals 1 auf, d. h. die beiden Extrema des ersten Signals 1 und des zweiten Signals 2 werden praktisch zeitgleich erfasst. Die Kenntnis von Flussraten und Volumina ist nicht erforderlich, da beide Extrema zeitgleich erscheinen und somit eine Berechnung einer Zeitverschiebung zwischen den Signalen und eine Verschmelzung der Extrema der Signale entfallen kann.

Mithin ist es etwa in einer Entwicklungsphase der Mehrfachsensorvorrichtung möglich, anhand eines Kriteriums wie beispielsweise "Entfall einer Verschiebungsberechnung bei hinreichend zeitgleichem Auftreten von Sensorsignalextrema" durch Variation von Sensorpositionen der einzelnen Sensoren in der Mehrfachsensorvorrichtung jeweils das Auftreten von Extrema in zumindest zwei von Sensoren der Mehrfachsensorvorrichtung detektierten Signalen zu erfassen und sodann diejenigen Sensorpositionen als erfindungsgemäß räumlich nahe zueinander festzulegen, bei welchen die Extrema praktisch bzw. hinreichend zeitgleich auftreten. In anderen Worten ist eine Beabstandung der Sensoren in der Mehrfachsensorvorrichtung, und damit deren räumliche Nähe im Sinne der Erfindung, zahlenmäßig aus der Erfassung der Extrema in den Sensorsignalen ermittelbar, d. h. ist dann festlegbar erhalten und bestimmt, wenn die Extrema der betrachteten Signale hinreichend zeitgleich auftreten.

Ein weiterer Vorteil der erfindungsgemäß räumlich nahen Anordnung besteht darin, dass durch die Anordnung der Sensoren in unmittelbarer Nähe zueinander auf Schlauchverbindungen untereinander verzichtet werden kann. Schlauchverbindungen setzen in der Regel Schlaucholiven mit entsprechender Länge an den einzelnen Komponenten voraus, um die Schläuche dort sicher anbringen zu können. Solche Verbindungen bergen jedoch auch die Gefahr entstehender Toträume, die nur schwer desinfiziert werden können.

Bei gemäß dem vorliegenden Ausführungsbeispiel drei Sensoren (Leitfähigkeitszelle 8, Temperatursensor 9 und optischer Sensor 10) resultieren sechs Möglichkeiten der Anordnung derselben in Flussrichtung in der Abführleitung 7. Jedoch kann es vorteilhaft sein die Leitfähigkeitszelle 8 und die Temperatursonde 9 nicht voneinander zu trennen, sodass diese jedenfalls direkt nebeneinander liegen, und den optischen Sensor 10 entweder unmittelbar vor der Leitfähigkeitszelle 8 oder unmittelbar nach der Temperatursonde 9 anzuordnen. Bevorzugt wird hierbei der optische Sensor 10 neben der Temperatursonde 9 angeordnet, da Sensoren, deren Daten zu fusionieren sind, vorteilhafterweise in unmittelbarer Nähe zueinander positioniert werden sollten.

Wie vorstehend in Übereinstimmung mit dem vorliegenden Ausführungsbeispiel beschrieben wurde, können die in der Gehäuseeinheit 11 aufgenommenen einzelnen Sensoren 8, 9 und 10 einen optischen Sensor zur Bestimmung der Extinktion und eine temperaturkompensierende bzw. temperaturkompensierte Leitfähigkeitszelle ausbilden. Die Erfindung ist jedoch nicht hierauf beschränkt, sondern es können darüber hinaus auch weitere Sensoren aufgenommen sein.

Dialysemaschinen beispielsweise besitzen in der Abführleitung 7 in der Regel einen optischen Sensor zur Detektion eines möglichen Blutlecks. Bevorzugt können daher ein solcher Blutleckdetektor und der optische Sensor 10 miteinander kombiniert werden, da beide Sensoren auch bereits verwandte Bauelemente (beispielsweise Lichtquellen, Fotodetektoren und dergleichen) aufweisen.

Wie vorstehend beschrieben wurde, sind bei einer Mehrfachsensorvorrichtung für ein medizinisches Gerät zumindest eine erste Sensoreinrichtung 8, 9 und zumindest eine zweite Sensoreinrichtung 10 an einer Fluid führenden Leitungsverbindung 7, entlang welcher die Sensoreinrichtungen 8, 9, 10 zumindest eine Größe aus einem strömenden Fluid detektieren, derart in vorbestimmter räumlicher Nähe zueinander angeordnet, dass vorbestimmte Signalanteile in Ausgaben jeweils der ersten und der zweiten Sensoreinrichtung praktisch zeitgleich auftreten und erfassbar sind. In einem Verfahren zur Festlegung einer räumlichen Nähe bei einer solchen Mehrfachsensorvorrichtung werden Positionen der einzelnen Sensoren 8, 9, 10 in der Mehrfachsensorvorrichtung variiert und wird jeweils das Auftreten vorbestimmter Signalanteile in zumindest zwei von den einzelnen Sensoren 8, 9, 10 detektierten Signalen erfasst, und werden diejenigen Positionen als Positionen der räumlichen Nähe festgelegt, bei welchen die vorbestimmten Signalanteile praktisch zeitgleich auftreten.

Es versteht sich, dass die Erfindung nicht auf das vorstehend beschriebene Ausführungsbeispiel beschränkt ist, sondern Änderungen, Modifikationen und äquivalente Anordnungen im Rahmen des anspruchsgemäß definierten Schutzumfangs ebenfalls von der Erfindung umfasst sind.

## Patentansprüche

1. Mehrfachsensorvorrichtung für ein medizinisches Gerät, bei der
zumindest eine erste Sensoreinrichtung (8, 9) und zumindest eine n-te Sensoreinrichtung (10) an einer Fluid führenden Leitungsverbindung (7), entlang welcher die Sensoreinrichtungen (8, 9, 10) zumindest eine Größe aus einem strömenden Fluid detektieren, in vorbestimmter räumlicher Nähe zueinander angeordnet sind,
eine maximale Distanz zwischen der ersten Sensoreinrichtung (8, 9) und der n-ten Sensoreinrichtung (10) durch eine vorbestimmte maximale Zeitverzögerung zwischen der Erfassung einer ersten Signaländerung eines ersten Signals der ersten Sensoreinrichtung (8, 9) und der Erfassung einer zu der ersten Signaländerung korrespondierenden Signaländerung eines n-ten Signals der n-ten Sensoreinrichtung (10) bei einer vorbestimmten Flussrate durch die Fluid führende Leitungsverbindung (7) bestimmt ist, und
die räumliche Nähe der ersten Sensoreinrichtung (8, 9) und der n-ten Sensoreinrichtung (10) derart vorbestimmt ist, dass bei der vorbestimmten Flussrate in dem ersten Signal der ersten Sensoreinrichtung (8, 9) und in dem n-ten Signal der n-ten Sensoreinrichtung (10) jeweils zueinander korrespondierende Signaländerungen bei parallel gleichzeitiger Erfassung beider Signale innerhalb der vorbestimmten maximalen Zeitverzögerung quasi zeitgleich auftreten,
**dadurch gekennzeichnet, dass**
die vorbestimmte maximale Zeitverzögerung 1 Sekunde beträgt und die vorbestimmte Flussrate in einem Bereich zwischen 100 ml/min und 800 ml/min liegt.

2. Mehrfachsensorvorrichtung nach Anspruch 1, bei der
das medizinische Gerät eine Maschine für extrakorporale Blutbehandlung ist;
die zumindest eine erste Sensoreinrichtung eine nicht-optische Sensoreinrichtung (8, 9) ist;
die zumindest eine n-te Sensoreinrichtung eine optische Sensoreinrichtung (10) ist; und
die Fluid führende Leitungsverbindung eine Abführleitung für verbrauchte Dialysierflüssigkeit (7) der Maschine für extrakorporale Blutbehandlung ist.

3. Mehrfachsensorvorrichtung nach Anspruch 2, bei der:
eine Gehäuseeinrichtung (11) an der Abführleitung für verbrauchte Dialysierflüssigkeit (7) dazu angeordnet ist, die zumindest eine nicht-optische Sensoreinrichtung (8, 9) und die zumindest eine n-te optische Sensoreinrichtung (10) in der vorbestimmten räumlichen Nähe zueinander aufzunehmen;
die zumindest eine nicht-optische Sensoreinrichtung (8, 9) dazu angeordnet ist, ein erstes Signal aus einer in der Abführleitung für verbrauchte Dialysierflüssigkeit (7) strömenden verbrauchten Dialysierflüssigkeit zu erfassen und auszugeben;
die zumindest einen-te optische Sensoreinrichtung (10) dazu angeordnet ist, ein n-tes Signal aus der in der Abführleitung für verbrauchte Dialysierflüssigkeit (7) strömenden verbrauchten Dialysierflüssigkeit zu erfassen und auszugeben; wobei
die räumliche Nähe der ersten Sensoreinrichtung (8, 9) und der n-ten Sensoreinrichtung (10) entlang der Abführleitung für verbrauchte Dialysierflüssigkeit (7) derart vorbestimmt ist, dass in dem ersten Signal der ersten Sensoreinrichtung (8, 9) und in dem n-ten Signal der n-ten Sensoreinrichtung (10) jeweils zueinander korrespondierende Signalabschnitte bei parallel gleichzeitiger Erfassung beider Signale praktisch zeitverschiebungsfrei auftreten.

4. Mehrfachsensorvorrichtung nach einem der vorangehenden Ansprüche, bei der
die zumindest eine erste Sensoreinrichtung (8, 9) eine Leitfähigkeitszelle (8) zur Bestimmung einer Leitfähigkeit verbrauchter Dialysierflüssigkeit und eine Temperatursonde (9) zur Messung einer Temperatur verbrauchter Dialysierflüssigkeit und zur Temperaturkompensation der Leitfähigkeitszelle (8) beinhaltet; und
die zumindest eine n-te Sensoreinrichtung (10) zumindest einen optischen Sensor (10) in Form zumindest einer Fotodiode und vorzugsweise zwei Fotodetektoren zur Bestimmung einer Absorptionseigenschaft der verbrauchten Dialysierflüssigkeit beinhaltet.

5. Mehrfachsensorvorrichtung nach Anspruch 4, bei der die Leitfähigkeitszelle (8) und die Temperatursonde (9) zu einer gemeinsamen Einheit innerhalb der Vorrichtung zusammengefasst und verbaut sind.

6. Mehrfachsensorvorrichtung nach Anspruch4 oder 5, bei der eine unmittelbare Nähe der Temperatursonde (9) zu dem optischen Sensor (10) derart vorbestimmt ist, dass eine von der Temperatursonde (9) erfasste Temperatur zur Korrektur temperaturabhängiger Effekte innerhalb des optischen Sensors (10) heranziehbar ist.

7. Mehrfachsensorvorrichtung nach einem der vorangehenden Ansprüche, bei der die vorbestimmte räumliche Nähe derart festgelegt ist, dass innerhalb der Mehrfachsensorvorrichtung die jeweiligen Sensoreinrichtungen zumindest untereinander schlauchverbindungsfrei angeordnet und aufgenommen sind.

8. Mehrfachsensorvorrichtung nach einem der Ansprüche 4 bis 6, bei der der optische Sensor (10) unmittelbar vor der Leitfähigkeitszelle (8) oder unmittelbar nach der Temperatursonde (9) angeordnet ist, und bevorzugt vor der Temperatursonde (9) angeordnet ist.

9. Mehrfachsensorvorrichtung nach einem der Ansprüche 4 bis 6 oder 8, bei der der optische Sensor (10) mit einem Blutleckdetektor kombiniert angeordnet ist.

## Claims

1. A multi-sensor device for a medical apparatus in which at least one first sensor unit (8, 9) and at least one n-th sensor unit (10) are arranged at a fluid-guiding line connection (7) along which the sensor units (8, 9, 10) detect at least one variable from a flowing fluid in predetermined proximity to each other,
a maximum distance between the first sensor unit (8, 9) and the n-th sensor unit (10) is determined by a predetermined maximum time delay between the detection of a first signal change of a first signal of the first sensor unit (8, 9) and the detection of a signal change of an n-th signal of the n-th sensor unit (10), wherein said signal change corresponds to the first signal change at a predetermined flow rate through the fluid-guiding line connection (7), and
the proximity of the first sensor unit (8, 9) and of the n-th sensor unit (10) is predetermined in such manner that, at the predetermined flow rate, in the first signal of the first sensor unit (8, 9) and in the n-th signal of the n-th sensor unit (10), respectively corresponding signal changes are occurring practically free from time shifts when both signals are simultaneously detected in parallel within the predetermined maximum time delay,
**characterized in that**
the predetermined maximum time delay is 1 and the predetermined flow rate lies between 100 ml/min and 800 ml/min

2. The multi-sensor device according to claim 1, wherein
the medical apparatus is a machine for extracorporeal blood treatment;
the at least one first sensor unit (8, 9) is a non-optical sensor unit;
the at least one n-th sensor unit (10) is an optical sensor unit; and
the fluid-guiding line connection (7) is a drain line for used dialysis fluid (7) of the machine for extracorporeal blood treatment.

3. The multi-sensor device according to claim 2, wherein:
a housing unit (11) is arranged at the drain line for used dialysis fluid (7) to accommodate the at least one non-optical sensor unit (8, 9) and the at least one n-th optical sensor unit (10) in the predetermined proximity to each other;
the at least one non-optical sensor unit (8, 9) is arranged to detect and to output a first signal from the used dialysis fluid flowing in the drain line for used dialysis fluid (7);
the at least one n-th optical sensor unit (10) is arranged to detect and to output an n-th signal from the used dialysis fluid flowing in the drain line for used dialysis fluid (7);wherein
the proximity of the first sensor unit (8, 9) and of the n-th sensor unit (10) along the drain line for used dialysis fluid (7) is predetermined in such manner that in the first signal of the first sensor unit (8, 9) and in the n-th signal of the n-th sensor unit (10) signal portions corresponding to each other are occurring practically free from time shifts when both signals are simultaneously detected in parallel.

4. The multi-sensor device according to one of the preceding claims, wherein
the at least one first sensor unit (8, 9) includes a conductivity cell (8) for determining conductivity of used dialysis fluid and a temperature probe (9) for measuring a temperature of used dialysis fluid and for temperature compensation of the conductivity cell (8); and
the at least one n-th sensor unit (10) includes at least one optical sensor (10) in the form of at least one photodiode and preferably two photodetectors for determining an absorption characteristic of the used dialysis fluid.

5. The multi-sensor device according to claim 4, wherein the conductivity cell (8)
and the temperature probe (9) are combined and installed in a joint unit within the device.

6. The multi-sensor device according to claim 4 or 5, wherein
a direct vicinity of the temperature probe (9) to the optical sensor (10) is predetermined such that a temperature detected by the temperature probe (9) can be used to correct temperature-dependent effects within the optical sensor (10).

7. The multi-sensor device according to one of the preceding claims, wherein
the predetermined proximity is defined in such manner that within the multi-sensor device the respective sensor units are arranged and accommodated at least among each other free from tube connections.

8. The multi-sensor device according to one of claims 4 to 6, wherein the optical sensor (10) is arranged directly upstream of the conductivity cell (8) or directly downstream of the temperature probe (9) and is preferably arranged upstream of the temperature probe (9).

9. The multi-sensor device according to one of claims 4 to 6 or 8, wherein the optical sensor (10) is arranged to be combined with a blood leakage detector.

## Revendications

1. Dispositif à plusieurs capteurs pour un appareil médical, dans lequel
au moins un premier appareil à capteur (8, 9) et au moins un nième appareil à capteur (10) sont disposés au niveau d'un raccordement de conduite conductrice de fluide (7), le long duquel les appareils à capteurs (8, 9, 10) détectent au moins une grandeur issue d'un fluide en écoulement à une proximité spatiale prédéterminée les uns par rapport aux autres,
une distance maximale entre le premier appareil à capteur (8, 9) et l'énième appareil à capteur (10) est déterminée par une temporisation maximale prédéterminée entre l'acquisition d'une première modification de signal d'un premier signal du premier appareil à capteur (8, 9) et l'acquisition d'une modification de signal, correspondant à la première modification de signal, d'un énième signal de l'énième appareil à capteur (10) à un débit prédéterminé à travers le raccordement de conduite conductrice de fluide (7), et
la proximité spatiale du premier appareil à capteur (8, 9) et de l'énième appareil à capteur (10) est prédéterminée de telle sorte qu'à un débit prédéterminé dans le premier signal du premier appareil à capteur (8, 9) et dans l'énième signal de l'énième appareil à capteur (10) des modifications de signal correspondant respectivement les unes aux autres lors d'une acquisition simultanée parallèle des deux signaux au sein de la temporisation maximale prédéterminée surviennent quasiment en même temps,
**caractérisé en ce que**
la temporisation maximale prédéterminée dure 1 seconde et le débit prédéterminé se situe dans une plage entre 100 ml/min et 800 ml/min.

2. Dispositif à plusieurs capteurs selon la revendication 1, dans lequel
l'appareil médical est une machine pour un traitement extracorporel du sang ;
l'au moins un premier appareil à capteur est un appareil à capteur non optique (8, 9) ;
l'au moins un énième appareil à capteur est un appareil à capteur optique (10) ; et
le raccordement de conduite conductrice de fluide est une conduite d'évacuation pour un liquide de dialyse usagé (7) de la machine pour traitement extracorporel du sang.

3. Dispositif à plusieurs capteurs selon la revendication 2, dans lequel :
un appareil formant boîtier (11) est agencé au niveau de la conduite d'évacuation pour liquide de dialyse usagé (7) pour loger l'au moins un appareil à capteur non optique (8, 9) et l'au moins un énième appareil à capteur optique (10) l'un par rapport à l'autre à la proximité spatiale prédéterminée ;
l'au moins un appareil à capteur non optique (8, 9) est agencé de manière à acquérir et émettre un premier signal à partir d'un liquide de dialyse usagé s'écoulant dans la conduite d'évacuation pour liquide de dialyse usagé (7) ;
l'au moins un énième appareil à capteur optique (10) est agencé pour acquérir et émettre un énième signal à partir du liquide de dialyse usagé s'écoulant dans la conduite d'évacuation pour liquide de dialyse usagé (7) ; dans lequel
la proximité spatiale du premier appareil à capteur (8, 9) et de l'énième appareil à capteur (10) le long de la conduite d'évacuation pour liquide de dialyse usagé (7) est prédéterminée de telle sorte que dans le premier signal du premier appareil à capteur (8, 9) et dans l'énième signal de l'énième appareil à capteur (10) des sections de signal correspondant respectivement entre elles lors d'une acquisition simultanée parallèle des deux signaux apparaissent pratiquement sans décalage temporel.

4. Dispositif à plusieurs capteurs selon l'une quelconque des revendications précédentes, dans lequel
l'au moins un premier appareil à capteur (8, 9) inclut une cellule de conductivité (8) pour la détermination d'une conductivité de liquide de dialyse usagé et une sonde de température (9) pour la mesure d'une température de liquide de dialyse usagé et pour une compensation de température de la cellule de conductivité (8) ; et
l'au moins un énième appareil à capteur (10) inclut au moins un capteur optique (10) sous la forme d'au moins une photodiode et de préférence deux photodétecteurs pour la détermination d'une propriété d'absorption du liquide de dialyse usagé.

5. Dispositif à plusieurs capteurs selon la revendication 4, dans lequel la cellule de conductivité (8) et la sonde de température (9) sont réunies et constituées en une unité commune au sein du dispositif.

6. Dispositif à plusieurs capteurs selon la revendication 4 ou 5, dans lequel une proximité directe de la sonde de température (9) et du capteur optique (10) est prédéterminée de telle sorte qu'une température acquise par la sonde de température (9) peut être mise à contribution pour la correction d'effets dépendants de la température au sein du capteur optique (10).

7. Dispositif à plusieurs capteurs selon l'une quelconque des revendications précédentes, dans lequel la proximité spatiale prédéterminée est définie de telle sorte qu'au sein du dispositif à plusieurs capteurs les appareils à capteurs respectifs sont disposés et logés au moins sans raccord de tuyau les uns en dessous des autres.

8. Dispositif à plusieurs capteurs selon l'une quelconque des revendications 4 à 6, dans lequel le capteur optique (10) est disposé directement en amont de la cellule de conductivité (8) ou directement en aval de la sonde de température (9) et de préférence en aval de la sonde de température (9).

9. Dispositif à plusieurs capteurs selon l'une quelconque des revendications 4 à 6 ou 8, dans lequel le capteur optique (10) est disposé combiné avec un détecteur de fuites de sang.
